# EUROPÄISCHE PATENTSCHRIFT

(11) **EP 1 289 406 B1**
(45) Veröffentlichungstag und Bekanntmachung des Hinweises auf die Patenterteilung: **20.10.2004**
(21) Anmeldenummer: 01921235.6
(22) Anmeldetag: 21.03.2001
(51) Int. Cl.: A61B 3/113

(54) **VORRICHTUNG ZUM KOPFFESTEN POSITIONIEREN VON MESSEINRICHTUNGEN, INSBESONDERE EINRICHTUNGEN ZUM MESSEN VON AUGENBEWEGUNGEN**
DEVICE FOR POSITIONING MEASURING DEVICES, ESPECIALLY DEVICES FOR MEASURING EYE MOVEMENTS, IN A MANNER THAT IS FIXED TO THE HEAD OF A TEST PERSON
DISPOSITIF POUR POSITIONNER DES DISPOSITIFS DE MESURE, NOTAMMENT DE DISPOSITIFS POUR MESURER LE MOUVEMENT DE L'OEIL, DE MANIERE FIXE SUR LA TETE D'UN PATIENT

(30) Priorität: 07.04.2000 DE 20006333 U
(43) Veröffentlichungstag der Anmeldung: 12.03.2003
(73) Patentinhaber: Chronos Vision GmbH, 12247 Berlin (DE)
(72) Erfinder: CLARKE, Andrew, A., 14163 Berlin (DE)
(74) Vertreter: Willems, Volker
(86) Internationale Anmeldenummer: PCT/DE2001/001126
(87) Internationale Veröffentlichungsnummer: WO 2001/076464

(56) Entgegenhaltungen:
- DE-U- 29 822 047

## Beschreibung

Die Erfindung betrifft eine Vorrichtung zum kopffesten Positionieren von Messeinrichtungen, insbesondere Einrichtungen zum Messen von Augenbewegungen, am Kopf eines Probanden mit einem Gestell, das aus mindestens einem etwa parallel zu den Augen ausgerichteten, ungefähr an die Kopfform des Probanden angepassten Halteträger und einem senkrecht zum Halteträger ausgerichteten Stirnträger gebildet ist, mindestens einem seitlich zum Gestell angeordneten einstellbaren Bildsensor, dessen Objektiv zu mindestens einem Infrarotspiegel ausgerichtet ist, mit das Auge beleuchtenden, seitlich des Objektives angeordneten, Infrarotlicht ausstrahlenden Beleuchtungselementen.

Bei diversen Messverfahren in der Neurologie, Ophthalmologie und Ergonometrie sind häufig Messgeräte am Kopf des Patienten bzw. Probanden zu fixieren. Das kopffeste Positionieren der Messgeräte ist besonders wichtig, wenn Augenbewegungen mittels elektronischer Bildverarbeitung gemessen werden sollen.

Aus den DE 296 03 944 und DE 298 22 047 ist eine Vorrichtung zur Erfassung von Augenbewegungen in Form einer Brille, einer Maske oder eines Helmes bekannt, bei der für jedes Auge mindestens ein einstellbarer Bildaufnehmer und mindestens ein unbeweglicher Spiegel vorgesehen ist.
Mit dieser bekannten Lösung kann die Infrarotkamera in Abhängigkeit des Augenabstandes individuell auf den Probanden durch selbsthemmende Kippbewegungen eingestellt werden. Fixiert wird diese bekannte Brille durch ein Kopfband, dessen Länge verstellbar ist.
Eine solche Befestigung hat den Nachteil, dass ein Verrutschen der Messgeräte während der Messung nicht sicher auszuschließen ist.
Mit diesem Stand der Technik gelingt es ferner nicht, die Messgeräte getrennt auf den individuellen Augenabstand bzw. auf die Geradeaus-Blickrichtung des jeweiligen Auges, d.h. unilateral, einzustellen.

Bei diesem Stand der Technik liegt der Erfindung die Aufgabe zugrunde, die Messgeräte kopffest zu positionieren, ein bequemes Tragen für den Probanden zu erreichen und zugleich eine korrektere Justierung der Messgeräte an eine anatomische Referenzlinie vor dem jeweiligen Auge zu gewährleisten.

Diese Aufgabe wird durch eine Vorrichtung der eingangs genannten Gattung mit den kennzeichnenden Merkmalen des Anspruches 1 gelöst.
Vorteilhafte Ausgestaltungen der Vorrichtung sind den Unteransprüchen entnehmbar.

Die Erfindung zeichnet sich vor allem dadurch aus, dass mit der individuell geformten Gesichtmaske eine kopffeste Positionierung der Messgeräte möglich wird. Durch die Aufteilung des Visiers in zwei Visierhälften entsteht die Möglichkeit, jede Visierhälfte individuell entsprechend den vorliegenden Gegebenheiten einzustellen und die Messgeräte zu justieren.

Weitere Vorteile und Einzelheiten ergeben sich aus der nachfolgenden Beschreibung unter Bezugnahme auf die beigefügten Zeichnungen.

Die Erfindung soll nachstehend an einem Ausführungsbeispiel näher erläutert werden.

Es zeigt:
- Fig. 1: eine Seitenansicht der erfindungsgemäßen Vorrichtung,
- Fig. 2: eine Draufsicht der erfindungsgemäßen Vorrichtung und

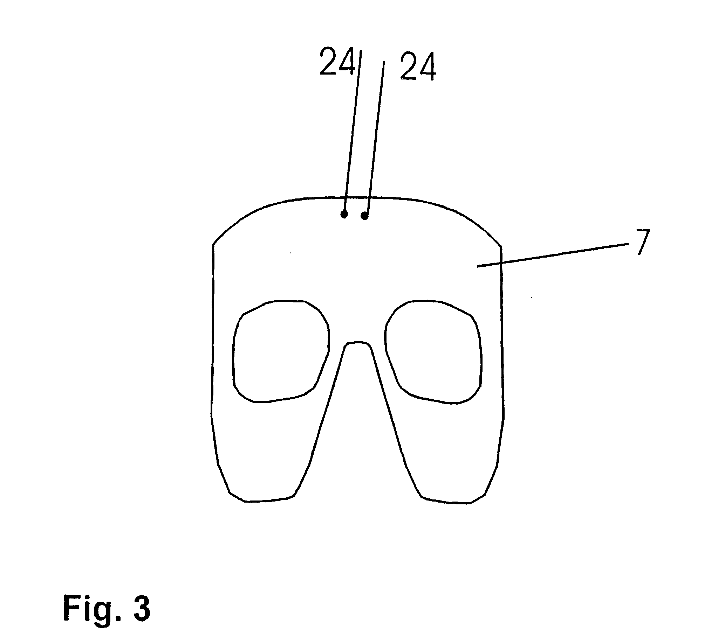

- Fig. 3: eine Draufsicht auf die Gesichtsmaske mit Festpunkten zur reproduzierbaren Befestigung am Stirnträger.

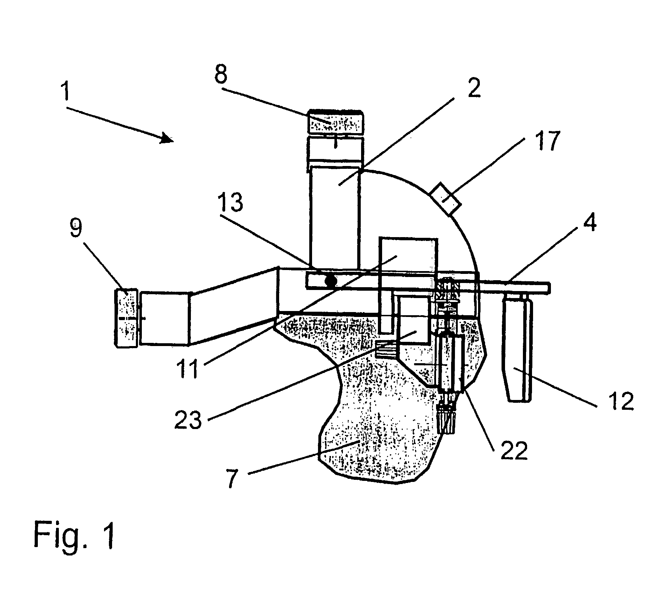

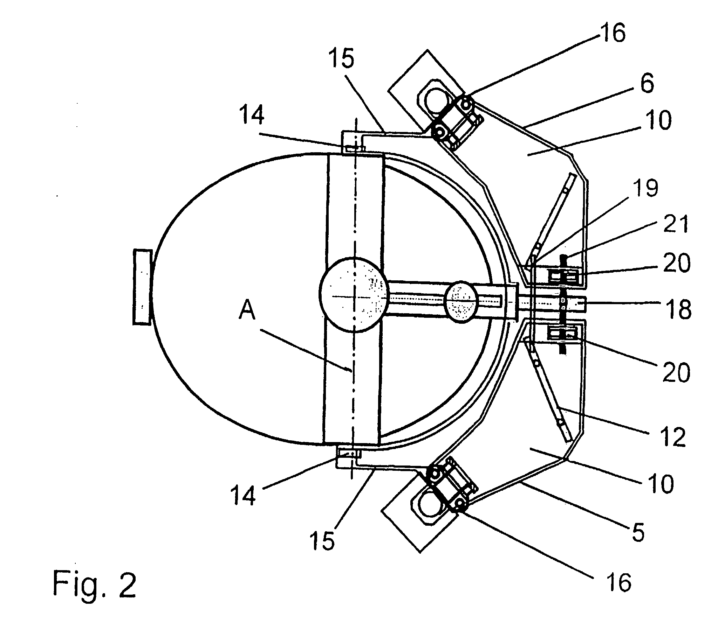

Die erfindungsgemäße Vorrichtung besteht im wesentlichen, wie in Fig. 1 und 2 gezeigt, aus einem Gestell **1,** das sich aus einem an die Kopfform angepassten Halteträger **2** und einem senkrecht vom Halteträger **2** ausgehenden Stirnträger **3** zusammensetzt, einem Visier **4**, das aus zwei die Messgeräte tragenden Visiereinheiten **5** und **6** gebildet ist, und aus einer Gesichtsmaske **7**. Halteträger **2** und Stirnträger **3** werden durch zwei Schrauben **8** und **9** an die individuelle Kopfform des Probanden angepasst. Die Gesichtsmaske **7** wird vor der Messung vom Gesicht des Probanden abgenommen. Zweckmäßigerweise verwendet man für die Herstellung der Gesichtsmaske **7** einen Thermoplastzuschnitt, den man erwärmt, auf das Gesicht des Probanden auflegt und das Gesicht entsprechend abformt. Sie hat etwa eine Dicke von 2 mm.
Nach dem Abkühlen kann die fertige Gesichtsmaske abgenommen werden. Bei Bedarf ist es aber auch möglich, auf vorgefertigte Gesichtsmasken zurückzugreifen, die von Standardgesichtsformen hergestellt sind.
Jede Visiereinheit **5** bzw. **6** hat einen Plattenteil **10**, an dem der Bildsensor **11** mit Objektiv **23**, die Beleuchtungselemente **22** und der Infrarotlichtspiegel **12** gehalten sind.
An beiden Enden **13** des Halteträgers **2** befindet sich ein Gelenk **14**, an dem ein bügelartig gebogener Federarm **15** drehbar gelagert ist. Der Federarm **15** verbindet jeweils beide Enden **16** der Visiereinheiten **5** bzw. **6**, so dass beide Visiereinheiten **5** bzw. **6** gemeinsam um die durch die Gelenke **14** gelegte Achse **A** vertikal verschwenkt werden können. Dies ermöglicht, die gesamte Visierebene mit den dazugehörigen Messgeräten auf eine anatomische Referenzlinie einzustellen. Typischerweise bietet es sich hier an, die sogenannte "Frankfurter Horizontale" -definiert als die Verbindungslinie zwischen der oberen Kante des äußeren Gehörganges und der unteren Kante der Augenhöhle, zu verwenden. Die jeweilige Schwenkhöhe lässt sich durch eine Stellschraube **17** arretieren, die in einer länglichen Aussparung des Stirnträgers **3** geführt wird.
Vom Stirnträger **3** ragt ein etwa mittig zwischen den Augen des Probanden gelegener Steg **18** auf, durch den eine Führungstange **19** senkrecht hindurchgeführt ist. Der Führungsstange **19** sind entsprechende im Plattenteil **10** eingebrachte Bohrungen zugeordnet. Im Plattenteil **10** befindet parallel zum Steg **18** eine Aussparung, in der eine Rändelschraube **20** auf einer senkrecht zum Steg **18** liegenden Gewindestange **21** drehbar befestigt ist. Die Gewindestange **21** durchdringt den Plattenteil **10**. Wird die Rändelschraube **20** gedreht, bewegt sich der Plattenteil **10** entweder vom Steg **18** weg oder auf diesen zu. Dies hat eine laterale Verschiebung der gesamten Visiereinheit **5** bzw. **6** mit allen daran befestigten Messgeräte zur Folge. Der Verschiebeweg wird durch den gebogenen Federarm **15** aufgenommen.
Durch zwei in der Gesichtsmaske **7** eingelassenen Stifte **24**, die in zwei entsprechende Aufnahmen, beispielsweise Bohrungen, im Stirnträger **3** passen, wird die reproduzierbare Positionierung der gesamten Vorrichtung erreicht.

Aufstellung der verwendeten Bezugszeichen
- Gestell: 1
- Halteträger: 2
- Stirnträger: 3
- Visier: 4
- Visiereinheiten: 5, 6
- Gesichtsmaske: 7
- Schrauben: 8, 9
- Plattenteil: 10
- Bildsensor: 11
- Infrarotlichtspiegel: 12
- Ende von 2: 13
- Gelenk: 14
- Federarm: 15
- Ende von 5,6: 16
- Stellschraube: 17
- Steg: 18
- Führungsstange: 19
- Rändelschraube: 20
- Gewindestange: 21
- Beleuchtungselemente: 22
- Objektiv: 23
- Festpunkte, Stifte: 24
- Schwenkachse: A

## Patentansprüche

1. Vorrichtung zum kopffesten Positionieren von Messeinrichtungen, insbesondere Einrichtungen zum Messen von Augenbewegungen, am Kopf eines Probanden, mit einem Gestell, das aus mindestens einem etwa parallel zu den Augen ausrichtbaren, ungefähr an die Kopfform des Probanden anpassbaren Halteträger und einem senkrecht zum Halteträger ausgerichteten Stirnträger gebildet ist, mindestens einem seitlich zum Gestell angeordneten einstellbaren Bildsensor, dessen Objektiv zu mindestens einem Infrarotspiegel ausgerichtet ist, mit zum Beleuchten des Auges, seitlich des Objektives angeordneten, Infrarotlicht ausstrahlenden Beleuchtungselementen,
mit folgenden Merkmalen:
a) eine von dem Gesicht des jeweiligen Probanden vor der Messung abformbare und beim Messvorgang tragbare Gesichtsmaske **(7)**, die zum reproduzierbaren Positionieren der Messeinrichtungen mit Festpunkten **(24)** versehen ist und
b) ein vom Gestell **(1)** gehaltenes Visier **(4),** das in zwei deckungsgleiche, seitenverkehrt zueinander angeordnete, jeweils den Bildsensor **(11)**, die Infrarotspiegel **(12)**, Objektiv **(23)** und die Beleuchtungselemente **(22)** aufnehmende Visiereinheiten **(5;6)** geteilt ist, die einzeln in einer Horizontalebene parallelogrammartig gegenüber der Geradeausblickrichtung des Probanden lateral verschieb- und einstellbar und **(5;6)** gemeinsam um eine Achse **(A)** vertikal verschwenkbar angeordnet sind.

2. Vorrichtung nach Anspruch 1, **dadurch gekennzeichnet, dass** die Gesichtsmaske **(7)** aus einer frei formbaren Masse, vorzugsweise Niedertemperatur-Thermoplaste, besteht.

3. Vorrichtung nach Anspruch 1, **dadurch gekennzeichnet, dass** die Festpunkte **(24)** aus mindestens zwei von der Gesichtsmaske **(7)** gehaltenen Stiften, Clips oder Druckknöpfen gebildet sind, denen entsprechende Aufnahmen im Stirnträger **(3)** zugeordnet sind.

4. Vorrichtung nach Anspruch 1 bis 3, **dadurch gekennzeichnet, dass** Dicke der Gesichtmaske etwa 1,5 mm bis 3 mm, vorzugsweise 2 mm beträgt.

5. Vorrichtung nach Anspruch 1, **dadurch gekennzeichnet, dass** der Stirnträger **(3)** einen von der Stirn wegragenden Steg **(18)** als Bezugsachse für die Lateralverschiebung aufweist.

6. Vorrichtung nach Anspruch 1 und 5, **dadurch gekennzeichnet, dass** jede Visiereinheit **(5;6)** einen Plattenteil **(10)** aufweist, an dem der Bildsensor vertikal verstellbar und der Infrarotspiegel in einer festgelegten Winkelstellung zum Bildsensor **(11)** angeordnet ist.

7. Vorrichtung nach einem oder mehreren der vorherigen Ansprüche 1 bis 6, **dadurch gekennzeichnet, dass** beide Visiereinheiten **(5;6)** einerseits an ihrem den Bildsensor tragenden Enden miteinander durch einen bügelartig gebogenen Federarm **(15)** verbunden sind, der im Stirnträger **(3)** geführt und in am jeweiligen Ende des Halteträgers **(2)** angeordneten Gelenken **(14)** drehbar gelagert ist, und andererseits die dem Steg **(18)** zugewandten Enden der Visiereinheiten **(5;6)** durch eine im Steg **(18)** gehaltene Stange **(19)** geführt und durch eine im jeweiligen Plattenteil **(10)** auf einer Gewindestange **(21)** drehbar angeordnete Rändelschraube **(20)** so lateral verschiebbar ist, dass der jeweilige Verschiebeweg von dem Federarm **(15)** aufgenommen wird.

8. Vorrichtung nach einem oder mehreren der vorherigen Ansprüche 1 bis 7, **dadurch gekennzeichnet, dass** die Gelenke **(14)** auf einer etwa interaural gelegenen Schwenkachse **(A)** angeordnet sind.

9. Vorrichtung nach Anspruch 1, **dadurch gekennzeichnet, dass** der Stirnträger **(3)** aus zwei Teilen besteht, wobei ein Teil mit einer länglichen Aussparung und der andere Teil mit einer in der Aussparung geführten Stellschraube **(17)** zum Arretieren der Stellposition versehen ist.

10. Vorrichtung nach Anspruch 1, **dadurch gekennzeichnet, dass** der Halteträger **(2)** und der Stirnträger **(3)** mit einer Polsterung versehen ist.

## Claims

1. A device for fixed positioning of measuring devices, particularly devices for measuring eye movements, to the head of a subject, comprising a frame made up of at least one retaining holder alignable approximately parallel with the eyes and adapted to the subject's head shape and a forehead holder aligned at right angles to the retaining holder, at least one adjustable image sensor disposed at the side of the frame and with its objective aligned with at least one infrared mirror, with eye-illuminating elements disposed at the side of the objective and radiating infrared light, comprising:
a) a face mask (7) adapted to the shape of the subject's face before the measurement and wearable during the measuring process and provided with fixed points (24) for reproducible positioning of the measuring devices and
b) an eyepiece (4) held by the frame (1) and divided into two sighting units (5; 6) identical in shape, disposed in laterally inverted relationship to one another and each holding the image sensor (11), the infrared mirror (12), the objective (23) and the illumination elements (22), the sighting units being individually movable and adjustable laterally in a horizontal plane in a parallelogram relative to the straight-ahead direction of view of the subject and vertically pivotable around a common axis (A).

2. A device according to claim 1, **characterised in that** the face mask (7) is made of a freely shapable material, preferably low-temperature thermoplastic.

3. A device according to claim 1, **characterised in that** the fixed points (24) are in the form of at least two pins, clips or press-buttons held by the face mask (7) and associated with corresponding recesses in the forehead holder (3).

4. A device according to claims 1 to 3, **characterised in that** the thickness of the face mask is about 1.5 to 3 mm, preferably 2 mm.

5. A device according to claim 1, **characterised in that** the forehead holder (3) has a reference axis for the lateral movement in the form of a web (18) projecting from the forehead.

6. A device according to claims 1 and 5, **characterised in that** each sighting unit (5; 6) has a plate part (10) on which the image sensor is vertically adjustable and the infrared mirror is disposed at a fixed angle relative to the image sensor (11).

7. A device according to one or more of the preceding claims 1 to 6, **characterised in that** the two sighting units (5; 6) are on the one hand connected to one another at their ends bearing the image sensor by a curved spring arm (15) which is guided in the forehead holder (3) and rotatably mounted in joints (14) disposed at the respective end of the retaining holder (2), and on the other hand the ends of the sighting units (5; 6) facing the web (18) are guided by a rod (19) held in the web (18) and are laterally movable by a knurled screw (20) disposed for rotation on a screwthreaded rod (21) in the respective plate part (10) so that the respective distance travelled is absorbed by the spring arm (15).

8. A device according to one or more of the preceding claims 1 to 7, **characterised in that** the joints (14) are disposed on a swivel axis (A) situated approximately between the ears.

9. A device according to claim 1, **characterised in that** the forehead holder (3) is in two parts, wherein one part is formed with an elongate recess and the other part comprises an adjusting screw (17) guided in the recess to lock in the adjusted position.

10. A device according to claim 1, **characterised in that** the retaining holder (2) and the forehead holder (3) are padded.

## Revendications

1. Dispositif pour positionner des dispositifs de mesure de manière fixe par rapport à une tête, notamment des dispositifs pour mesurer des mouvements oculaires, au niveau de la tête d'un volontaire, avec un support constitué par au moins un support de retenue orientable parallèlement aux yeux et étant à peu près ajustable à la forme de la tête du volontaire, et un support de front orienté perpendiculairement au support de retenue, au moins un capteur d'image ajustable disposé au coté du support, dont l'objectif est orienté vers au moins un miroir infrarouge, et des éléments d'éclairage émettant de la lumière infrarouge, disposés à coté de l'objectif pour éclairer l'oeil, avec les caractéristiques suivantes :
a) Un masque de visage **(7)** pouvant être mis en forme pour le visage du volontaire respectif avant la mesure, et porté lors du processus de mesure, étant pourvu de points fixes **(24)** pour le positionnement reproductible des dispositifs de mesure, et
b) Un viseur **(4)** tenu par le support **(1)**, qui est divisé en unités de viseur **(5 ; 6)** de même surfaces et disposées inversement l'une par rapport à l'autre, comportant respectivement le capteur d'image **(11)**, les miroirs infrarouges **(12)**, l'objectif **(23)** et les éléments d'éclairage **(22)**, étant individuellement latéralement déplaçable et ajustable dans un plan horizontal en parallélogramme par rapport au regard vers l'avant du volontaire, et disposées pour être ensemble pivotées verticalement autour d'un axe **(A)**.

2. Dispositif selon la revendication 1, **caractérisé en ce que** le masque de visage **(7)** consiste en une masse pouvant librement être mise en forme, de préférence un plastique thermique déformable à basse température.

3. Dispositif selon la revendication 1, **caractérisé en ce que** les points fixes **(24)** sont formés par au moins deux broches tenues par le masque de visage **(7)**, pinces ou boutons pressoirs auxquels correspondent des contreparties respectives sur le support de front **(3)**.

4. Dispositif selon les revendications 1 à 3, **caractérisé en ce que** l'épaisseur du masque se situe par exemple entre 1,5 mm et 3 mm, de préférence à 2 mm.

5. Dispositif selon la revendication 1, **caractérisé en ce que** le support de front **(3)** comporte une arête **(18)** partant du front comme axe de référence pour le déplacement latéral.

6. Dispositif selon les revendications 1 et 5, **caractérisé en ce que** chaque unité de viseur **(5 ; 6)** comprend une partie de plaque **(10)** sur laquelle le capteur d'image est disposé pour être ajusté verticalement et le miroir infrarouge dans un angle fixe par rapport au capteur d'image **(11)**.

7. Dispositif selon une ou plusieurs des revendications précédentes 1 à 6, **caractérisé en ce que** les deux unités de viseur **(5 ; 6)** sont d'une part reliées l'une à l'autre à leurs extrémités portant le capteur d'image par un bras à ressort **(15)** tordu en branche qui passe dans le support de front **(3)** et qui est fixé pour pouvoir tourner à des articulations **(14)** disposées aux extrémités respectives du support de retenu **(2)**, et d'autre part les extrémités des unités de viseur **(5 ; 6)** orientées vers l'arête sont guidées par une barre **(19)** retenue dans l'arête **(18)** et peuvent être déplacées latéralement par une vis moletée **(20)** dans la partie de plaque **(10)** respective, étant vissée sur une barre de vis **(21)** de telle façon que le chemin de déplacement est absorbé par le bras à ressort **(15)**.

8. Dispositif selon une ou plusieurs des revendications précédentes 1 à 7, **caractérisé en ce que** les articulations **(14)** sont disposées sur un axe de pivotement **(A)** sensiblement reliant les oreilles.

9. Dispositif selon la revendication 1, **caractérisé en ce que** le support de front **(3)** est constitué de deux parties, une partie comportant une niche longitudinale et l'autre partie une vis de réglage **(17)** introduite dans la niche pour arrêter la position d'ajustement.

10. Dispositif selon la revendication 1, **caractérisé en ce que** le support de retenue **(2)** et le support de front **(3)** sont pourvus d'un capitonnage.
